# EUROPEAN PATENT SPECIFICATION

(11) **EP 1 915 211 B1**
(45) Date of publication and mention of the grant of the patent: **16.09.2009**
(21) Application number: 05764356.1
(22) Date of filing: 07.07.2005
(51) Int. Cl.: B01L 3/00

(54) **METHOD AND DEVICE FOR ACOUSTIC MANIPULATION OF PARTICLES, CELLS AND VIRUSES**
VERFAHREN UND VORRICHTUNG ZUR AKUSTISCHEN MANIPULATION VON PARTIKELN, ZELLEN UND VIREN
PROCEDE ET DISPOSITIF DE MANIPULATION ACOUSTIQUE DE PARTICULES, CELLULES ET VIRUS

(43) Date of publication of application: 30.04.2008
(73) Proprietor: Fraunhofer-Gesellschaft zur Förderung der angewandten Forschung e.V., 80686 München (DE)
(72) Inventor: LEMOR, Robert, 66111 Saarbrücken (DE); GÜNTHER, Christian, 66111 Saarbrücken (DE); FUHR, Günter Prof. Dr., 13187 Berlin (DE); WIKLUND, Martin, S-11253 Stockholm (SE); HERTZ, Hans, S-18279 Stocksund (SE)
(74) Representative: Gagel, Roland
(86) International application number: PCT/EP2005/007355
(87) International publication number: WO 2007/006322

(56) References cited:
- US-A1- 2004 066 703
- PATENT ABSTRACTS OF JAPAN vol. 2003, no. 12, 5 December 2003 (2003-12-05) & JP 2004 340820 A (OLYMPUS CORP), 2 December 2004 (2004-12-02)

## Description

### Field of the invention

The present invention relates to a method and device for non-intrusively manipulating suspended particles and/or cells and/or viruses, which are supplied to a micro-chamber or to a micro-channel of a substrate, in accordance with claims 1 or 17.

A non-intrusive separation, positioning, concentration or other manipulation of particles and/or cells and/or viruses in micro-channels or micro-chambers is required in various technical fields including applications in bio-technology and cell-biology.

### Background of the invention

It is known that suspended particles or cells can be non-intrusively handled in micro-channels and micro-chambers by several methods. The corresponding micro-systems comprise substrates with channel structures through which a suspension fluid flows with the particles to be manipulated. As a rule the cross section area of these channel structures is rectangular, with the width of the top and bottom channel walls, i.e. the walls of the channel which in the operating position of the micro-system are at the top and at the bottom, being greater than the height of the lateral channel walls. According to a known method of non-intrusive manipulation, which is known for example from WO 00/00293, the suspended particles or cells are manipulated by dielectrophoretic forces. To this end, microelectrodes are affixed to the channel walls, with high frequency electrical fields being applied to said microelectrodes. Under the influence of the high frequency electrical fields, based on negative or positive dielectrophoresis, polarization forces are generated in the suspended particles or cells. These polarization forces can lead to a repulsion from the electrodes and, acting in combination with flow forces in the carrying fluid, allow a manipulation of the particles in the channel. The term manipulation in the present patent application is used to describe all kinds of controllable external influence on the particles which cause a defined movement or holding of the particles or cells which would not occur without this external influence. Examples for such a manipulation are positioning, concentrating, guiding or separating particles in the micro-chamber or micro-channel.

Conventional micro-systems have disadvantages in relation to the effectiveness of generating the polarization forces. This relates in particular to the stability and longevity of the microelectrodes as well as to a limited ability of generating force gradients within the channel structure. These disadvantages are in particular linked to the electrode bands which are formed over comparatively long distances in the channel. The longer an electrode band, the longer a particle flowing past is in the sphere of influence of the electrode band. Consequently, the effectiveness of the respective microelectrode or the field barrier generated by this microelectrode increases. However, long electrode bands are also more susceptible to malfunction. Faults in workmanship or mechanical loads can cause interruptions of these bands which lead to electrode failure. Due to these disadvantages the application of fluidic micro-systems with dielectrophoretic particle manipulation has been limited to the guidance of particles in the channel structure or to the deflection of particles from a given flow.

Another technology known for manipulation of suspended particles is based on an optical trapping mechanism. With so called laser tweezers it is possible to hold or move particles in suspension with micrometer accuracy. Disadvantages of this technology are the required high external apparatus which hinders miniaturization,' and the energy deposition into the material in the focal spot.

In recent years acoustic radiation forces for manipulating suspended particles or cells have come into use. It is known that particles or cells can be manipulated by standing and/or stationary wave acoustic fields. One of the problems arising with this acoustic manipulation is the coupling efficiency of the acoustic waves into the inner volume of the micro-channels or micro-chambers, which often have only a small height compared with their lateral dimensions. F. Peterson et al., "Separation of Lipids from Blood Utilizing Ultrasonic Standing Waves in Micro-Fluidic Channels", Analyst, 2004, 129, pp. 938-43, propose the application of the ultrasonic waves vertically from the top surface or from the back surface, in the present description also called bottom surface, of the substrate to the inner volume. To this end the acoustic transducer is mounted directly above or below the channel on the top surface or on the back surface of the substrate, i.e. the microchip. The same approach is also described in WO 02/072235 A1 (Laurell et al.) and in E. Nilsson et al., "Acoustic Control of Suspended Particles in Micro-Fluidic Chips", Lab Chip, 2004, pp. 131-135. In these documents the physical effects of standing and/or stationary acoustic waves in the micro-channels or micro-chambers leading to the manipulation of the particles or cells are described in detail. These documents, therefore, are cited in the present patent application as background references with respect to the explanation and use of these physical effects.

The acoustic techniques proposed in the above documents, however, are nevertheless lacking an efficient coupling of energy into the channels. Furthermore, the control of the ultrasonic standing and/or stationary wave fields along the channels is very limited. The described acoustic setups also do not directly allow for transmission optical microscopy to observe the particles or cells in the channels during manipulation.

US 2004/0066703 discloses a device for non-intrusively manipulating suspended particles and/or cells and/or viruses.

### Summary of the invention

An object of the present invention is to provide a method and a device for non-intrusively manipulating suspended particles and/or cells and/or viruses, which allow a more efficient coupling of acoustic energy into the channels, a better control of the standing and/or stationary acoustic waves along the channels or chambers and the possibility of observation of the particles and/or cells and/or viruses by optical transmission microscopy during manipulation.

The object is achieved with the method and device according to present claims 1 and 17. Advantageous embodiments of the method and the device are the subject matter of the sub claims and/or disclosed in the subsequent description and examples.

The micro-chamber or micro-channel used in the present method and device has at least a bottom wall and lateral walls, optionally also a top wall, and is integrated in a substrate, also called chip, having a top and a bottom surface. The top and the bottom surface of the substrate represent the surfaces with the largest area of such a substrate, the top and bottom being related to the orientation of the substrate during the intended use. The outer surfaces of the optional top wall and the bottom wall of the micro-chamber or micro-channel form part of the top and bottom surface of the substrate as is known in the art. In the present method and device the acoustic waves are applied laterally to the inner volume of the micro-chamber or micro-channel. The term laterally means that the main propagation axis of the incident acoustic wave in the substrate has a lateral component, i.e. a component perpendicular to the surface normal of the top or bottom surface of the substrate. Preferably this lateral component, in the following also called horizontal component, is larger than the vertical component which is parallel to the surface normal.

In the present method and device the lower limit for this lateral component is preferably given by the requirement that the acoustic transducers have to be arranged outside of a straight optical path through said top wall, said inner volume and said bottom wall, wherein said optical path allows optical transmission microscopy of the manipulated particles in the inner volume. Therefore, this optical path is not a single line but has also lateral dimensions providing an optical duct or window through the optional top wall, the inner volume and the bottom wall in order to allow said transmission microscopy.

Preferably, the acoustic transducers for lateral application of the acoustic waves are arranged such that they do not occlude the channel or chamber, not even partially, in top view or bottom view of the substrate.

The following description and examples for the reason of simplification only refer to micro-channels and to the manipulation of particles. It is expressly stated that the description and examples in the same manner can be applied to micro-chambers in case of micro-channels and to the manipulation of cells and/or viruses in addition to or instead of particles.

In the invention, the acoustic waves are launched into the substrate and inner volume by means of acoustic refractive elements mounted with one end face on the top and/or bottom surface of the substrate. The acoustic transducers are mounted on the other end face of the refractive elements. These refractive elements, which also could be seen as waveguides, are adapted, i.e. formed and/or adjusted, to allow the propagation of the acoustic waves in the substrate in a direction different from the surface normal direction of the top surface or bottom surface of said substrate. Refraction of acoustic waves takes place at the interface between two different materials due to different velocities of the acoustic waves within the two materials. In the present case such refraction occurs at least at the interface between the refractive element and the top layer or the substrate. The materials of the refractive element, of the substrate and of the optional top layer are adjusted such that a maximum amount of acoustic energy is coupled into the channel. Such a refractive element for coupling the acoustic power into the substrate and inner volume can be for example a prism shaped or wedge shaped element. The angle between the two end faces of the prism shaped or wedge shaped element can take any value as long as the above requirements are fulfilled. With these refractive elements the acoustic waves are coupled into the substrate from the top and bottom surfaces at an angle relative to the surface normal, resulting in a main propagation direction of the acoustic waves with a lateral component. With this technique the lateral coupling of the acoustic waves into said inner volume is possible through the top or bottom surface of the substrate without occluding the channel in top view or bottom view of the substrate.

In other words, a main idea of the present method and device is to couple the acoustic field into the inner volume of the micro-channel primarily horizontally, thereby increasing the coupling efficiency to relevant acoustic modes in the channel significantly and allowing for further optical investigation during manipulation through an optical transmission path in the vertical direction. The horizontal or lateral coupling refers to any geometric assembly of the acoustic transducers which allows the part of the micro-channel in which the particles are to be manipulated to be optically transparent in a vertical direction, i.e. the field of view not being obstructed by the acoustic transducers. In the case of commonly used micro-system designs as described above, this refers in particular to any geometric assembly, where the main propagation axis of the incident acoustic wave is primarily perpendicular or deviating only in a small angle from a perpendicular direction to the inner surfaces of the lateral walls of a rectangular or otherwise shaped micro-channel.

The present method and device are not limited to the generation of standing and/or stationary acoustic wave(s) by using the channel walls as a resonant cavity. It is also possible to generate a standing and/or stationary wave by interference of two acoustic waves traveling in opposite directions in the channel, e.g. by interference of two acoustic waves applied by two acoustic transducers arranged at opposite sides of the channel. Furthermore, in addition to the standing and/or stationary acoustic waves in the horizontal direction, also standing and/or stationary acoustic waves in the vertical direction of the channel can be generated with the same arrangement of acoustic transducers.

With the present method and device several different acoustic transducers can be placed at different positions along the micro-channel, thereby allowing different manipulation to be performed at different regions along the channel. Furthermore, by changing the frequency of the transducer, different node patterns in the channel can be created, allowing fast switching and, thus, manipulation. When using the channel walls as a resonant cavity for the acoustic wave, it is important that the resonator formed by the walls of the channel has the correct dimension with respect to the frequency of the acoustic wave. The horizontal coupling using refractive elements as coupling elements allows optical transmission microscopy to be performed at the time of manipulation, since no acoustic transducer covers the channel. The method is compliant with all-glass or glass-Si-glass structures allowing optical transmission microscopy in line. The method is also compliant with other materials of the substrate and of the top and bottom layer.

In the present method and device, when several acoustic transducers are arranged at several regions of the micro-channel, it is possible to use one single refractive element for the coupling of the acoustic waves of several transducers into the substrate and inner volume. In this case the several transducers are mounted side by side on said refractive element. It is also possible to provide for each acoustic transducer a separate refractive element. Furthermore some of the transducers can couple the acoustic waves to the substrate via refractive elements, wherein others may be attached directly to the side surfaces of the substrate. All combinations are possible depending on the intended effect of manipulation.

In a preferred embodiment of the proposed method and device acoustic manipulation is combined with dielectrophoretic manipulation on the same chip, i.e. in the same micro-channel. Examples and background for the technique of dielectrophoretic manipulation are described for example in WO 00/00293, which describes the technique of dielectrophoretic manipulation and appropriate electrode patterns used for this manipulation.

The two techniques of acoustic manipulation via standing and/or stationary acoustic waves and dielectrophoretic (DEP) manipulation can be used in a sequential arrangement in the micro-channel. The sequential arrangement is related to the flow direction of a laminar flow of the fluid in which the particles are suspended, or to the movement direction of these particles, which can be caused by centrifugal forces applied to the micro-system. Dielectrophoretic manipulation is preferably used downstream of a region of acoustic manipulation. In such an arrangement, acoustic manipulation can be used first to align the particles in one or several lines via a standing and/or stationary acoustic wave which is generated perpendicular to the flow or movement direction. And then, downstream of this region, DEP manipulation can be used to further manipulate, for example to trap, said pre-aligned particles with dielectrophoretic forces. When acoustic pre-alignment is performed in combination with the later on dielectrophoretic manipulation the exposure of the particles to electric fields is minimized. In this context it is also possible to alternate between regions for acoustic manipulation and regions for dielectrophoretic manipulation along the channel length.

In a further embodiment both techniques are applied in parallel. In this case acoustic manipulation and DEP manipulation are performed in overlapping regions of the micro-channel at the same time. The short range forces of DEP allow a precise positioning of the particles, wherein the acoustic forces keep them at a sufficient close range to the electrodes in one or two dimensions. It is evident that the two embodiments, i.e. the sequential and the parallel arrangement of means for both techniques, can be used in combination throughout the length of the micro-channel.

An advantage of the combination of DEP and acoustic manipulation is that less DEP forces are needed for manipulation, resulting in less potential damage of in particular cells or viruses. The flexibility of use of the two independent forces allows an accurate manipulation of the particles.

The proposed device for non-intrusively manipulating suspended particles comprises a substrate with at least one integrated micro-chamber or micro-channel, said micro-chamber or micro-channel having at least a bottom wall as well as lateral walls, and with at least one acoustic transducer for applying an acoustic wave from outside of said substrate to an inner volume of said micro-chamber or micro-channel. The acoustic transducer is arranged to apply said acoustic wave laterally to said volume. Preferably the acoustic transducer is arranged outside of a straight optical path through an optional top wall, said inner volume and said bottom wall. The geometric dimensions of the substrate and the micro-chamber or micro-channel are preferably the same as already known in the art in the field of such lab-on-a-chip devices. The present device, however, is not limited to these known dimensions.

The acoustic transducer can be any kind of transducer which is able to generate the necessary acoustic wave. An example for such a transducer is a piezoceramic plate, for example of PZT, which is able to emit acoustic waves in the required frequency range. Generally, the frequency range for the acoustic waves can vary between frequencies in the kHz up to the GHz range.

In one embodiment of such a device, the top wall and the bottom wall of the micro-channel are thin enough to allow optical transmission microscopy with a high numerical aperture for observing the manipulated particles in said micro-channel. The observation is possible since the acoustic transducers are arranged outside of the straight optical path needed for optical transmission microscopy.

In the present description and claims the word "comprising" or "comprises" does not exclude other elements or steps as well as an "a" or "an" does not exclude a plurality. Also any reference signs in the claims shall not be construed as limiting the scope of these claims.

### Brief description of the drawings

Exemplary embodiments of the proposed method and device are described in the following with reference to the accompanying drawings without limiting the scope of the claims. The drawings show:
- Fig. 1: a cross section of a micro-channel (or micro-chamber) with one (fig. 1a) or several (fig. 1b) acoustic waves being applied;
- Fig. 2: three examples of channel geometries of a micro-channel in a cross sectional view;
- Fig. 3: a top view and cross section of an exemplary substrate showing different geometries of micro-channels;
- Fig. 4: an example showing lateral application of the acoustic wave in two different manners according to the present invention;
- Fig. 5: an example of acoustic manipulation according to the present invention;
- Fig. 6: a further example of acoustic manipulation according to the present invention;
- Fig. 7: a further example of acoustic manipulation according to the present invention;
- Fig. 8: a further example of acoustic manipulation according to the present invention;
- Fig. 9: a further example of acoustic manipulation according to the present invention;
- Fig. 10: a further example of acoustic manipulation according to the present invention;
- Fig. 11: an example of a combination of acoustic manipulation and DEP manipulation according to the present invention;
- Fig. 12: a further example of acoustic manipulation according to the present invention;
- Fig. 13: a further example of combined acoustic an DEP manipulation according to the present invention;
- Fig. 14: a further example of acoustic manipulation according to the present invention; and
- Fig. 15: a further example of combined acoustic and DEP manipulation according to the present invention.

### Description of preferred embodiments

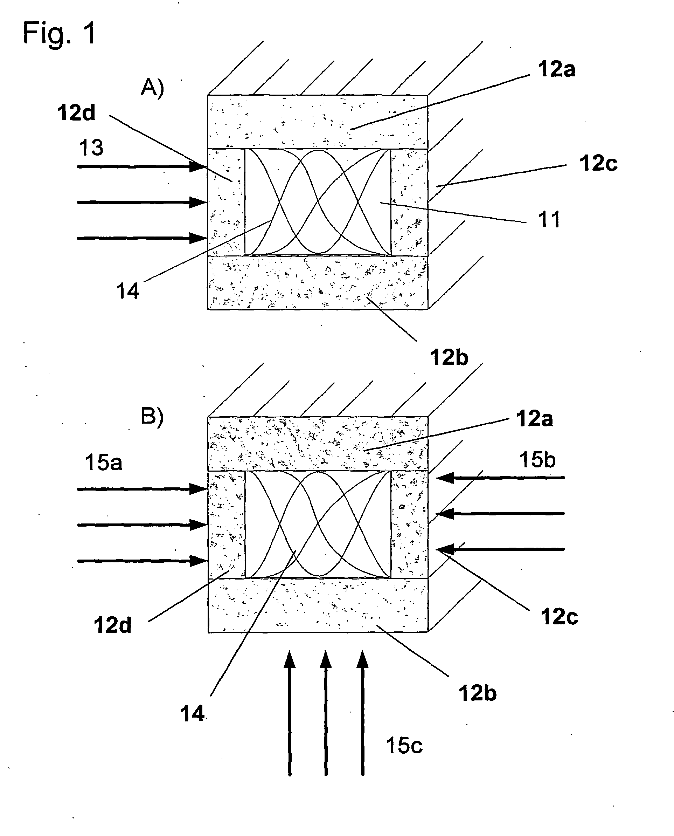

Figure 1 shows a schematical side view of a micro-channel 11 which is embedded in a surrounding material forming a top wall 12a, a bottom wall 12b and lateral walls 12c, 12d. An acoustic wave 13 is applied laterally to the inner volume of said micro-channel 11 as indicated in figure 1a. The inner surfaces of the walls 12a-12d of the micro-channel 11 are reflecting surfaces for the acoustic wave. With the distance of these inner surfaces adapted to the wavelength of the acoustic wave, a standing and/or stationary acoustic wave 14 forms in this micro-channel 11 as shown schematically in figure 1. The micro-channel 11 then serves as a resonating cavity for the acoustic wave to generate the standing and/or stationary wave 14. It is evident that the wavelength of the acoustic wave 13 also depends on the medium inside of this micro-channel 11, in particular of the type of fluid supplied to this micro-channel 11. As a rule, the frequency of the applied acoustic wave 13 is tuned appropriately to fulfil the resonance condition.

The standing and/or stationary acoustic wave 14 can also be generated through interference by applying acoustic waves 15a, 15b, 15c from different sides of the micro-channel 11. In this case, which is shown in figure 1b, a three dimensional stationary acoustic standing and/or stationary wave establishes, wherein the micro-channel 11 is not necessarily used as a resonant cavity.

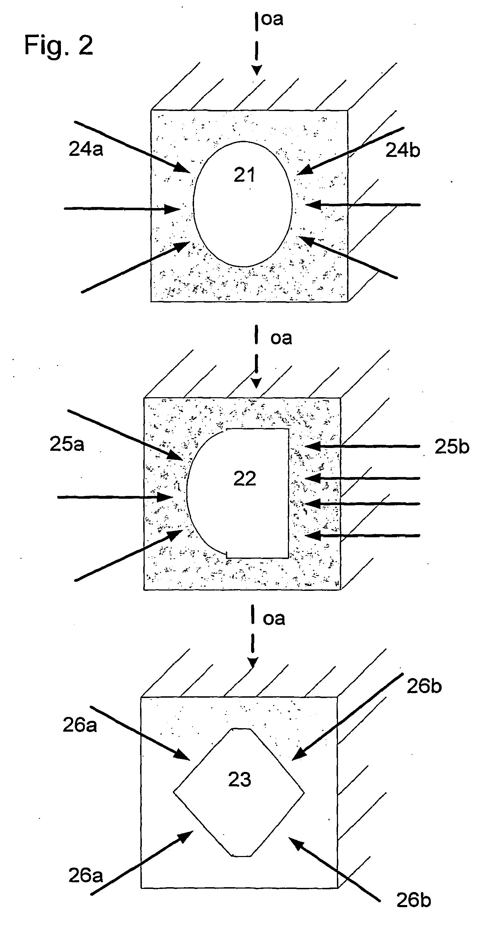

The cross sectional geometry of the micro-channel can differ from the rectangular shape and may have complex geometries as shown for example in the three sectional views of figure 2. This figure depicts different cross sectional geometries of micro-channels 21, 22 and 23. The optical axis (oa) is also indicated in this figure. This optical axis defines a straight optical path through the top wall, the inner volume and the bottom wall of the micro-channel, allowing the observation of the particles in said channel by transmission microscopy during the manipulation. The acoustic waves 24a, 24b, 25a, 25b, 26a and 26b are applied mainly horizontally so that the field of view with this optical axis is not obstructed by the acoustic transducers generating the acoustic waves. From figure 2 it is evident, that the channel geometry can be adapted to achieve an optimal resonance behavior for the frequencies of the acoustic waves.

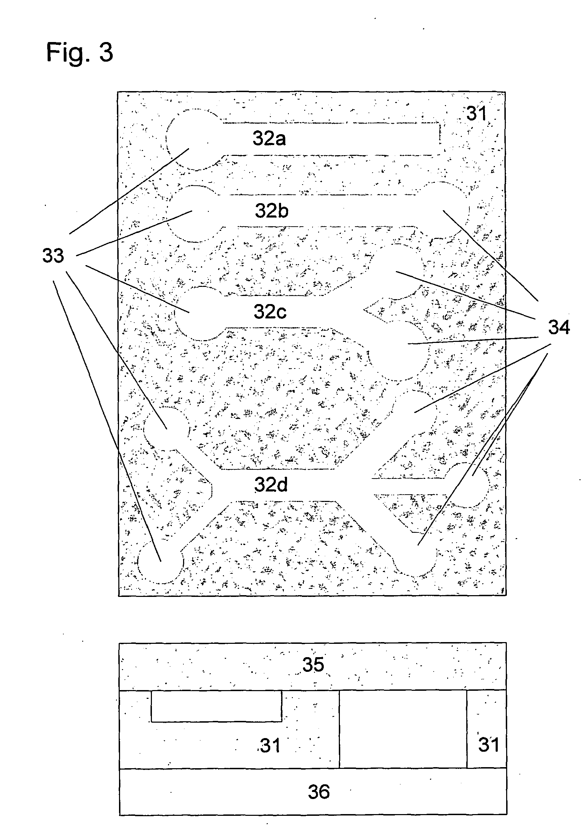

Figure 3 shows a carrier chip 31 which forms part of the substrate of the present device together with a top layer 35 and bottom layer 36. The carrier chip 31 can be made of transparent and/or non transparent glass or silicon or plastic and contains in this example one or more micro-channels 32a-32d for demonstration purposes. These channels (or chambers) are formed to transport or collect the suspensions of matter containing the particles to be manipulated. The channels are connected to one or more in- and outlets 33, 34 for supplying different suspensions and solutions. In order to build up the substrate, the carrier 31 is bonded on one or both sides to the top layer 35 and bottom layer 36, which are made of a transparent medium such as Pyrex-glass, which offers the possibility to observe the behavior in the channels with incident light and fluorescence microscopy. An example of a setup of such a substrate is a glass-silicon-glass sandwich which allows trans-illumination microscopy to be performed. It is also possible, that the top and bottom layers 35, 36 are optically transparent only in the regions of the channels.

As can be seen from this example which shows a top view and a cross sectional view of such a substrate or chip, a set of different geometries of the micro-channels can be used depending on the intended manipulation. Exemplary dimensions of the cross section of a micro-channel are a width of ca. 500 µm and a height of ca. 50 µm.

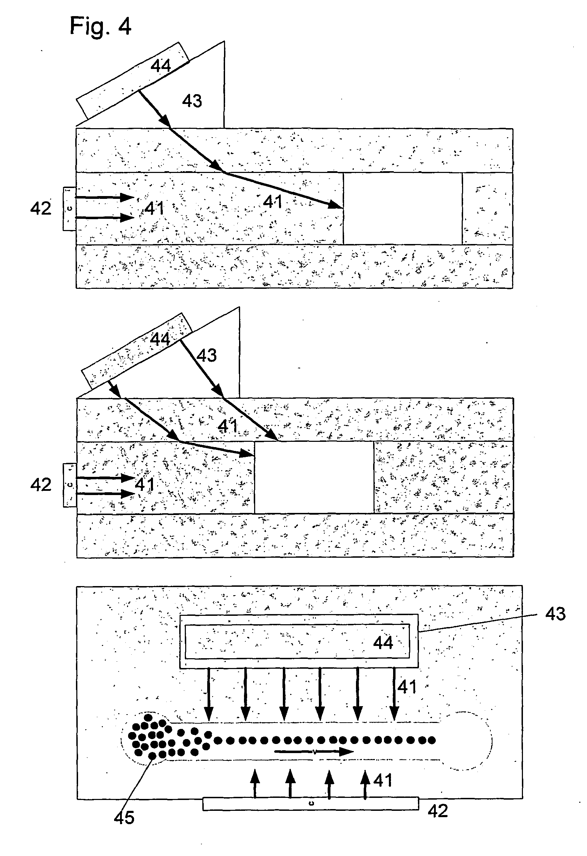

Fig. 4 shows an example of a substrate or chip in two cross sectional views and a top view indicating the arrangement of two transducers 42, 44 for laterally applying acoustic waves 41. Transducer 42 is mounted to the side surface of the substrate in order to apply the acoustic wave 41 laterally without any perpendicular component.

The acoustic wave 41 of the second acoustic transducer 44 is applied via an acoustic refractive element 43 (coupling element), in this case a transparent plastic coupling wedge. This refractive element 43 is mounted on the top surface of the substrate outside of the optical path through the top wall, inner volume and bottom wall of the micro-channel 46. The acoustic transducer 44 is directly mounted to the refractive element 43. Due to this coupling setup the acoustic wave 41 generated by said acoustic transducer 44 is applied displaced from the micro-channel and is refracted towards the micro-channel, resulting in a mainly lateral component of the propagation direction of this acoustic wave 41 in the top layer and substrate, which is schematically indicated in fig. 4.

The frequency of the acoustic wave 41 is selected to form a standing and/or stationary acoustic wave perpendicular to the flow of the particles along the micro-channel 46. By applying different acoustic waves from different sides, standing and/or stationary waves parallel and perpendicular to the flow can form. The flow direction in this example is indicated in the top view of the substrate of fig. 4. In this top view the particles 45 are shown as small dots. The applied acoustic wave 41 forms a standing and/or stationary acoustic wave perpendicular to the flow direction inside the channel 46. This standing and/or stationary acoustic wave has only one node in the present example. The particles 45 are aligned by this standing and/or stationary wave along the node to move with the flow velocity v in one line as indicated in the figure. With this setup, therefore, a contact free way of manipulation and treatment of micro- and nano-particles can be performed. By application of this one or several acoustic fields it is possible to hold, move or concentrate particles for further investigation, for example in biological cell science.

The particles may or may not move in such a channel for manipulation by the acoustic standing and/or stationary wave field. The applied frequency of the acoustic waves can vary with the acoustic properties of the suspension fluid and the geometry of the channel.

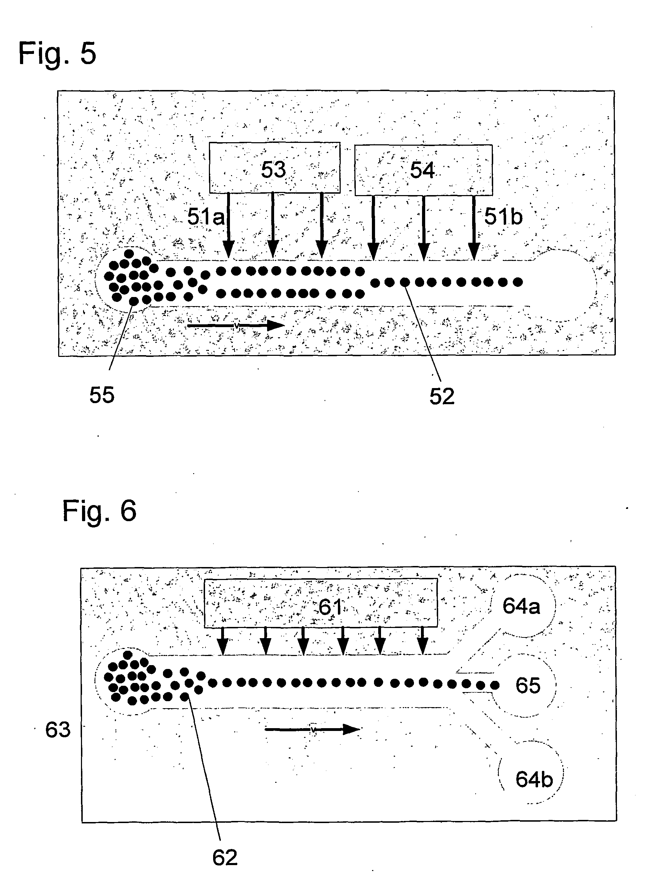

Fig. 5 shows a further example for acoustic manipulation of particles 55 according to the present invention. In this example, two acoustic transducers 53, 54 are arranged at different positions of the micro-channel. By applying acoustic waves 51a, 51b of different frequencies to the different regions of the channel, different resonant modes can be excited in the channel. The same effect can be achieved by applying the same frequency but changing the geometry of the channel in the different regions. In this example, the acoustic field 51a generated by acoustic transducer 53 forms a standing and/or stationary wave with two nodes, wherein the transducer 54 emits an acoustic wave 51b forming a standing and/or stationary acoustic wave with only one node in the channel. This results in the alignment of the particles 55 moving along the channel in two lines in the left side region and in one line in the right side region of this channel. If no turbulences appear in the flow, this sorting is permanent and stays after removing the influence of the ultrasonic transducers 53, 54.

Fig. 6 depicts another example showing the principle of concentration of flowing particles 62 with the help of an ultrasound coupling wedge transducer 61. A particle or cell suspension is fed to the system through an inlet 63 and flows through the channel with velocity v. The suspension fluid is taken out of the channel at the outlets 64a and 64b. The particle flow is concentrated in the middle of the channel by the standing and/or stationary acoustic wave and taken out of the channel over an additional outlet 65. Such a device can be used for the enrichment or concentration of particles in the same manner as a centrifuge.

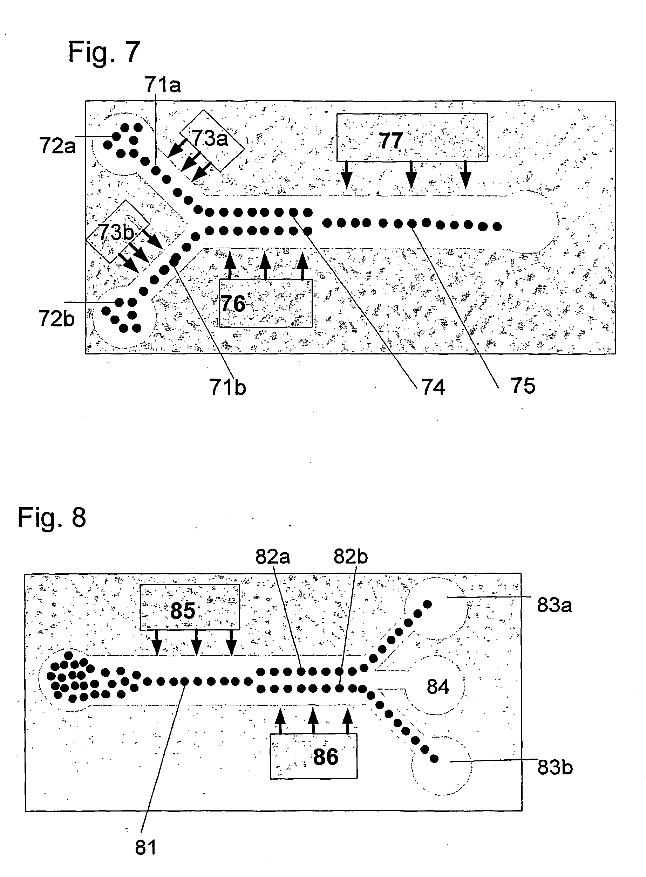

Fig. 7 shows a further example of the present invention and device in order to demonstrate the concept of mixing or controlled collocating two or more particle flows 71a, 71b coming from two or more particle sources 72a, 72b. The particle flows 71a, 71b are pre-aligned in this example through acoustic transducer setups 73a, 73b. In the common part of the channel system the two flows are previously sorted in several nodes 74 by means of acoustic transducer 76 and than mixed by switching to one single node 75 by means of acoustic transducer 77. This switching from two or more nodes 74 to one node 75 can be achieved by different frequencies of the acoustic waves in the two regions, as in the present case, or by changing the channel geometry between the two regions.

Fig. 8 shows a conceptual setup for sorting and separating streams with suspended particles or cells. The particles or cells are collected in a first node 81 by means of acoustic transducer 84 and then divided into two side nodes 82a, 82b by means of acoustic transducer 85. Due to the branching of the channel towards the outlets 83a, 83b, 84, the particles in the side nodes 82a, 82b are guided to different outlets 83a and 83b, which are also different from the outlet 84 for the central flow.

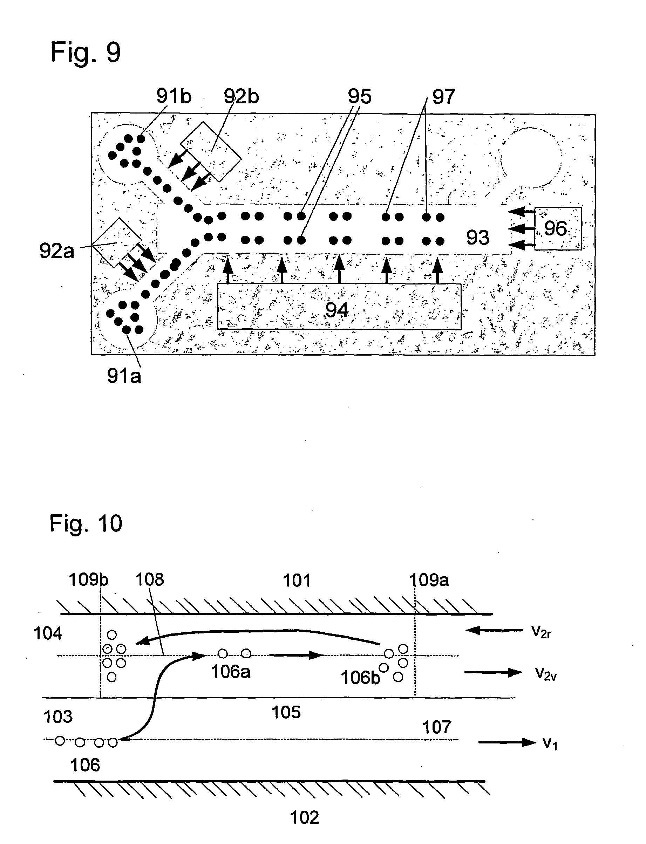

Fig. 9 shows a concept for portioning and treating small amounts of matter. The particles of one or more sources 91a, 91b are collected in one or more nodes by acoustic transducers 92a and 92b. The two particle streams are fed into chamber 93 in which standing and/or stationary acoustic wave fields are generated by transducers 94 and 96. The standing and/or stationary acoustic wave generated by transducer 94 splits the particle streams in two or more streams 95. The standing and/or stationary acoustic wave generated by transducer 96 parallel to the flow direction creates a grid of trapped particles 97. While trapping the particles perpendicular and parallel to the flow of the fluid, this transporting fluid can be changed which allows multiple applications such as washing, treating, staining and so on.

Fig. 10 shows an example in which particles are parked within the channel. In this and the following figures the acoustic transducers are not explicitly shown.

In the top view of the channel, a laminar flow of two solutions 103, 104 between lateral channel walls 101, 102 is shown. The vertical phase boundary 105 between the two solutions 103, 104 is schematically indicated as a straight line in fig. 10. In a first step, a standing and/or stationary acoustic wave is generated in this channel having a node 107 in which the particles of the first solution flowing with velocity v1 are collected. By change of the frequency of the acoustic wave these particles 106 are shifted through the phase boundary 105 into the second solution flowing with velocity v2. In this second solution the particles are also collected by a node 108 of a standing and/or stationary acoustic wave field. The particles are then additionally trapped in a region of the channel by generating a standing and/or stationary acoustic wave in the direction parallel to the flow. This further standing and/or stationary wave forms ultrasound barriers 109a, 109b which cannot be passed by the particles 106a, 106b, thus resulting in a parking of the particles. The particles 106b could be switched occasionally back into the main stream, i.e. the flow of the first solution, by changing the frequency of the applied acoustic waves. A disadvantage of this setup would be the need for a permanent acoustic field.

This permanent acoustic field can be avoided by switching the direction of flow of the second solution as schematically indicated with v₂ᵣ and v₂ᵥ in fig. 10. By switching this direction of flow and switching of the acoustic field, the particles can be transported between the ultrasound barriers 109a and 109b forward and backward. By periodically switching the flow direction, the particles can be hold in this region in the second solution until they have to be switched back to the first solution. This process can be optimized if the two solutions are not mixable.

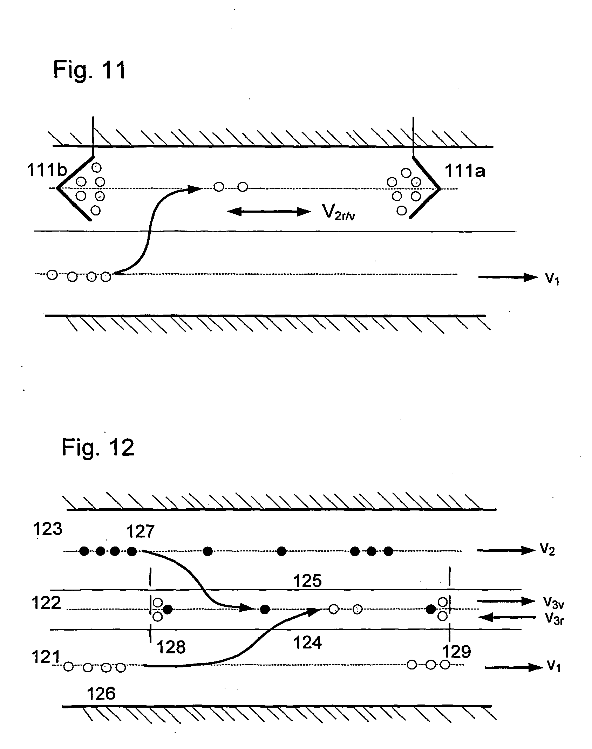

Fig. 11 shows a similar example for parking of particles. In this embodiment, additional electrodes 111a and 111b are arranged on the top and on the bottom layer of the micro-channel in the region of the flow of the second solution. By means of these electrodes 111b and 111a, a dielectrophoretic field is applied which forms a flow barrier for the particles. This flow barrier can be used instead of the ultrasound barrier of fig. 10.

Fig. 12 shows the formation of a flow in three segments 121, 122 and 123 with two phase boundaries 124, 125. In the same way as already explained with reference to figs. 10 and 11, particles of different types, indicated as black and light grey dots, can be transported to the middle of the channel by changing the acoustic standing and/or stationary waves. The barriers 128 and 129 in this example are also created by acoustic waves. For the inner flow, i.e. the flow of the inner segment 122, the trapping criteria has to be fulfilled as in the case of the upper flow in figs. 10 and 11. This trapping criteria means, that the forces applied by the laminar flow to the particles must be equal than the counter force generated by the standing and/or stationary acoustic wave or dielectrophoretic field at the corresponding barrier.

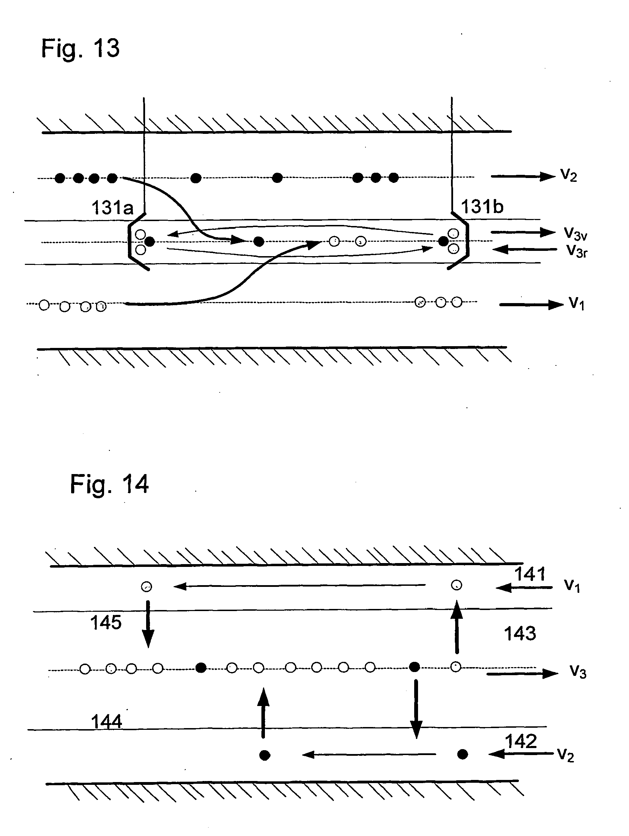

Fig. 13 shows the same concept as Fig. 12 with the difference, that in this example the barriers are generated by use of dielectrophoretic barrier electrodes 131a, 131b at the top and bottom walls of the channel.

Fig. 14 shows a micro-channel with three flowing solutions 141, 142, 143. The two outer solutions 141, 142 flow in opposite direction to the inner solution 143. The vertical phase boundaries 144 and 145 between these solutions are also indicated in fig. 14. By periodically switching the frequency of the standing and/or stationary acoustic wave, a central node of which is indicated in the middle of the flow of the inner solution 143, the particles in the middle can be switched to the solutions 141, 142 flowing in the opposite direction, and switched back into the inner flow. Therefore, also with this technique a parking loop is generated for the particles, as is evident from fig. 14.

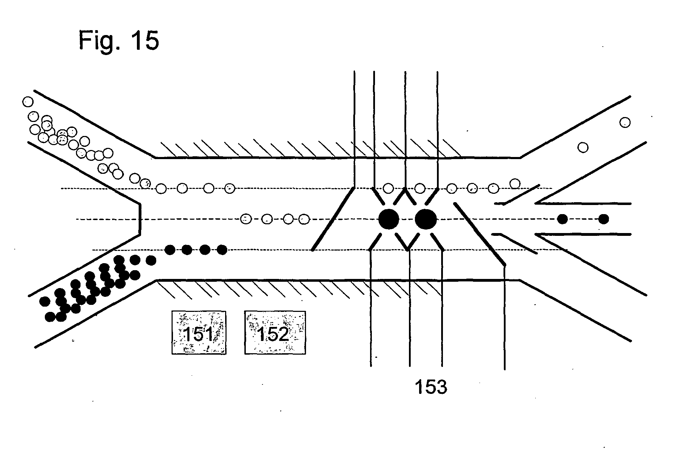

Fig. 15 shows a further example of the combined setup of acoustic and dielectrophoretic manipulation. The acoustic transducers 151, 152 allow a pre-alignment of one or more streams of particles in one or several nodes as indicated in the figure. The electrode setup 153 allows dielectrophoretic manipulation, in the present example for sorting the particles of different type to different channel branches. The two setups can either be arranged in a sequential manner, in which the regions of the micro-channel influenced by the two setups do not overlap, as is shown in the example of fig. 15. The two setups can also be arranged in parallel, in which case the regions of manipulation overlap in the micro-channel.

Generally, the regions of acoustic manipulation within the micro-channel can have dimensions ranging from some millimeters to some ten micrometers, in particular in combination with dielectrophoretic manipulation in regions of a similar dimension.

### List of Reference Signs

- 11: micro-channel
- 12a: top wall
- 12b: bottom wall
- 12c: lateral wall
- 12d: lateral wall
- 13: acoustic wave
- 14: standing and/or stationary acoustic wave
- 15a-c: acoustic waves
- 21-23: micro-channel
- 24a/b: acoustic wave
- 25a/b: acoustic wave
- 26a/b: acoustic wave
- 31: carrier chip
- 32a-d: micro-channel
- 33: inlet
- 34: outlet
- 35: top layer
- 36: bottom layer
- 41: acoustic wave
- 42: acoustic transducer
- 43: acoustic refractive element
- 44: acoustic transducer
- 45: particles or cells
- 46: micro-channel
- 51a/b: acoustic wave
- 52: micro_channel
- 53: acoustic transducer
- 54: acoustic transducer
- 55: particles or cells
- 61: acoustic transducer
- 62: particles or cells
- 63: inlet
- 64a/b: outlet
- 65: outlet
- 71a/b: particle flows
- 72a/b: particle sources
- 73a/b: acoustic transducers
- 74: several nodes
- 75: one node
- 76: acoustic transducer
- 77: acoustic transducer
- 81: central node
- 82a/b: side nodes
- 83a/b: side node outlets
- 84: central outlet
- 85: acoustic transducer
- 86: acoustic transducer
- 91a/b: particle sources
- 92a/b: acoustic transducers
- 93: main chamber
- 94: acoustic transducer
- 95: two or more particle streams
- 96: acoustic transducer
- 97: grid of trapped particles
- 101/102: lateral walls
- 103/104: two solutions
- 105: phase boundary
- 106: particles
- 106a/b: parked particles
- 107/108: nodes
- 109a/b: ultrasound barrier
- 111a/b: dielectrophoretic electrodes
- 121-123: different flows
- 124/125: phase boundaries
- 126/127: lateral walls
- 128/129: ultrasound barriers
- 131a/b: dielectrophoretic electrodes
- 141-143: different solutions or flows
- 144/145: phase boundaries
- 151/152: acoustic transducers
- 153: dielectrophoretic electrodes

## Claims

1. A method for non-intrusively manipulating suspended particles and/or cells and/or viruses, which are supplied to a micro-chamber or to a micro-channel (11) of a substrate, said micro-chamber or micro-channel (11) having at least a bottom wall (12b) as well as lateral walls (12c,12d), wherein at least one acoustic wave (41) is applied via at least one acoustic transducer (44) laterally to an inner volume of said micro-chamber or micro-channel (11), a frequency of said acoustic wave (41) being selected to generate a standing and/or stationary acoustic wave (14) in said volume,
**characterized in**
**that** said at least one acoustic wave (41) is applied via said at least one acoustic transducer (44) from outside of said substrate to the inner volume of said micro-chamber or micro-channel (11), wherein one or several of said acoustic wave(s) (41) are applied using an acoustic refractive element (43) between one or several of said acoustic transducer(s) (44) and said substrate, said refractive element (43) being formed to launch said acoustic wave(s) (41) into said substrate in a direction different from a surface normal direction of a top surface or a bottom surface of said substrate.

2. The method according to claim 1,
**characterized in that** said refractive element (43) is a wedge shaped or a prism shaped element.

3. The method according to claim 1 or 2,
**characterized in that** one or several of said refractive elements (43) are attached to the top surface and/or the bottom surface of said substrate.

4. The method according to any of claims 1 to 3,
**characterized in that** said at least one acoustic transducer (44) is arranged outside of a straight optical path through said volume and said bottom wall (12b).

5. The method according to any of claims 1 to 4,
**characterized in that** several acoustic waves are applied laterally via several acoustic transducers (44) at different regions and/or in different directions of said micro-chamber or micro-channel (11) in order to generate standing and/or stationary acoustic waves (14) in said volume, thereby allowing an identical or a different manipulation to be performed at different or same regions of the micro-chamber or micro-channel (11).

6. The method according to any of claims 1 to 5,
**characterized in that** the frequency of one or several of said acoustic wave(s) (41) is selected to form one or several nodes or antinodes of said standing and/or stationary acoustic wave(s) (14) between opposing lateral walls of said micro-chamber or micro-channel (11).

7. The method according to any of claims 1 to 6,
**characterized in that** the frequency of one or several of said acoustic wave(s) (41) is shifted in order to switch between different node patterns of the standing and/or stationary wave(s) (14).

8. The method according to any of claims 1 to 7,
**characterized in that** one or several of said acoustic wave(s) (41) are applied by arranging one or several of said acoustic transducer(s) (44) on side surfaces of said substrate.

9. The method according to any of claims 1 to 8,
**characterized in that** in combination with the manipulation by said standing and/or stationary acoustic wave(s) (14) a dielectrophoretic manipulation of said particles and/or cells and/or viruses in said micro-chamber or micro-channel (11) is performed.

10. The method according to claim 9,
**characterized in that** said dielectrophoretic manipulation is performed downstream of a region of manipulation by at least one of said standing and/or stationary acoustic wave(s) (14) with respect to a laminar flow of said particles and/or cells and/or viruses in said micro-chamber or micro-channel (11).

11. The method according to claim 10,
**characterized in that** said particles and/or cells and/or viruses are first aligned in one or several rows by said standing and/or stationary acoustic wave(s) (14) and then trapped by said dielectrophoretic manipulation.

12. The method according to claim 9,
**characterized in that** said dielectrophoretic manipulation and said manipulation by said standing and/or stationary acoustic wave(s) (14) are performed in overlapping regions of said volume at the same time.

13. The method according to any of claims 1 to 12,
**characterized in that** at least two parallel laminar flows of different fluids (103, 104) with said particles and/or cells and/or viruses are generated in said micro-channel or micro-chamber (11), wherein said particles and/or cells and/or viruses are switched from a first of said laminar flows to a second of said laminar flows by shifting or switching the frequency of one or several of said acoustic wave(s) (41) or by applying a dielectrophoretic force.

14. The method according to claim 13,
**characterized in that** said particles and/or cells and/or viruses which are switched to the second laminar flow, are trapped in a defined region in said second laminar flow by generating a barrier (109) for said particles and/or cells and/or viruses across said second laminar flow by means of a standing and/or stationary acoustic wave (14).

15. The method according to claim 13,
**characterized in that** said particles and/or cells and/or viruses which are switched to the second laminar flow, are trapped in a defined region in said second laminar flow by generating a barrier (109) for said particles and/or cells and/or viruses across said second laminar flow by means of dielectrophoretic forces.

16. The method according to claim 13,
**characterized in that** said particles and/or cells and/or viruses which are switched to the second laminar flow, are trapped in a defined region in said second laminar flow by periodically reversing a flow direction of said second laminar flow.

17. Device for non-intrusively manipulating suspended particles and/or cells and/or viruses, comprising a substrate with at least one integrated micro-chamber or micro-channel (11), said micro-chamber or micro-channel (11) having at least a bottom wall (12b) as well as lateral walls (12c,12d), and with at least one acoustic transducer (44) arranged to apply an acoustic wave (41) from outside of said substrate laterally to an inner volume of said micro-chamber or micro-channel (11),
**characterized in that** one or several of said acoustic transducers (44) are mounted on an acoustic refractive element (43) attached to a top and/or a bottom surface of said substrate, said refractive element (43) being formed to launch said acoustic wave(s) (41) into said substrate in a direction different from a surface normal direction of a top surface or a bottom surface of said substrate.

18. Device according to claim 17,
**characterized in that** said acoustic transducers (44) are arranged at different positions of said substrate.

19. Device according to claim 18,
**characterized in that** said acoustic transducers (44) are arranged at different sides of said substrate.

20. Device according to any of claims 17 to 19,
**characterized in that** said one or several of said acoustic transducers (44) are mounted to one or several side surfaces of said substrate.

21. Device according to any of claims 17 to 20,
**characterized in that** said refractive elements (43) are wedge shaped or prism shaped elements.

22. Device according to any of claims 17 to 21,
**characterized in that** said acoustic transducer (44) is arranged outside of a straight optical path through said volume and said bottom wall.

23. Device according to any of claims 17 to 22,
**characterized in that** in said bottom wall (12b) and/or in a top wall (12a) electrodes (111a,111b) are integrated allowing a dielectrophoretic manipulation of said particles and/or cells and/or viruses in said micro-chamber or micro-channel (11) in combination with the manipulation by standing and/or stationary acoustic wave(s) (14) generated by said one or several acoustic transducers (44).

24. Device according to claim 23,
**characterized in that** said electrodes (111a,111b) are arranged outside of one or several regions of manipulation by said standing and/or stationary acoustic wave(s) (14) with respect to a laminar flow of said particles and/or cells and/or viruses in said micro-chamber or micro-channel (11).

25. Device according to claim 23,
**characterized in that** said electrodes (111a,111b) are arranged in different regions of said micro-chamber or micro-channel (11) alternating with regions of manipulation by said standing and/or stationary acoustic wave(s) (14) on a longitudinal axis of said micro-chamber or micro-channel (11).

26. Device according to claim 23,
**characterized in that** said electrodes (111a,111b) are arranged in one or several regions influenced by said standing and/or stationary acoustic wave(s) (14) .

27. Device according to any of claims 23 to 26,
**characterized in that** said electrodes (111a,111b) are arranged to be able to form one or several traps for said particles and/or cells and/or viruses via dielectrophoretic manipulation.

28. Device according to any of claims 17 to 27,
**characterized in that** said substrate is of an optically transparent material at least in the region of a top wall (12a) and said bottom wall (12b) of said micro-chamber or micro-channel (11).

29. Device according to claim 28,
**characterized in that** said top wall (12a) and said bottom wall (12b) of said micro-chamber or micro-channel (11) have a thickness allowing the use of transmission microscopy with high numerical aperture for observation of said particles and/or cells and/or viruses in said volume.

## Patentansprüche

1. Verfahren zum nicht intrusiven Manipulieren suspendierter Teilchen und/oder Zellen und/oder Viren, welche einer Mikrokammer oder einem Mikrokanal (11) eines Substrats zugeführt werden, wobei die Mikrokammer oder der Mikrokanal (11) mindestens eine untere Wand (12b) sowie seitliche Wände (12c, 12d) aufweist, wobei mindestens eine Schallwelle (41) über mindestens einen Schallwandler (44) seitlich in ein inneres Volumen der Mikrokammer oder des Mikrokanals (11) eingebracht wird, wobei eine Frequenz der Schallwelle (41) so gewählt wird, dass in dem Volumen eine stehende und/oder stationäre Schallwelle (14) erzeugt wird,
**dadurch gekennzeichnet,**
**dass** die mindestens eine Schallwelle (41) über den mindestens einen Schallwandler (44) von außerhalb des Substrats in das innere Volumen der Mikrokammer oder des Mikrokanals (11) eingebracht wird, wobei eine oder mehrere der Schallwelle(n) (41) unter Verwendung eines Schallbrechungselements (43) zwischen einem oder mehreren der Schallwandler (44) und dem Substrat eingebracht werden, wobei das Brechungselement (43) so ausgebildet ist, dass die Schallwelle(n) (41) in einer Richtung in das Substrat eingekoppelt wird (werden), die sich von der Richtung einer Flächennormalen einer oberen Fläche oder einer unteren Fläche des Substrats unterscheidet.

2. Verfahren nach Anspruch 1, **dadurch gekennzeichnet, dass** es sich bei dem Brechungselement (43) um ein keilförmiges oder prismenförmiges Element handelt.

3. Verfahren nach Anspruch 1 oder 2, **dadurch gekennzeichnet, dass** eines oder mehrere der Brechungselemente (43) an der oberen Fläche und/oder der unteren Fläche des Substrats angebracht sind.

4. Verfahren nach einem der Ansprüche 1 bis 3, **dadurch gekennzeichnet, dass** der mindestens eine Schallwandler (44) außerhalb eines geraden Strahlenwegs durch das Volumen und die untere Wand (12b) hindurch angeordnet ist.

5. Verfahren nach einem der Ansprüche 1 bis 4, **dadurch gekennzeichnet, dass** mehrere Schallwellen über mehrere Schallwandler (44) in verschiedenen Bereichen und/oder verschiedenen Richtungen der Mikrokammer oder des Mikrokanals (11) seitlich eingebracht werden, um stehende und/oder stationäre Schallwellen (14) in dem Volumen zu erzeugen, wodurch ermöglicht wird, dass eine identische oder unterschiedliche Manipulation in unterschiedlichen oder denselben Bereichen der Mikrokammer oder des Mikrokanals (11) durchgeführt wird.

6. Verfahren nach einem der Ansprüche 1 bis 5, **dadurch gekennzeichnet, dass** die Frequenz von einer oder mehreren der Schallwelle(n) (41) so gewählt ist, dass ein oder mehrere Knoten oder Bäuche der stehenden und/oder stationären Schallwelle(n) (14) zwischen gegenüberliegenden seitlichen Wänden der Mikrokammer oder des Mikrokanals (11) gebildet werden.

7. Verfahren nach einem der Ansprüche 1 bis 6, **dadurch gekennzeichnet, dass** die Frequenz von einer oder mehreren der Schallwelle(n) (41) verschoben wird, um zwischen verschiedenen Knotenmustern der stehenden und/oder stationären Schallwelle(n) (14) umzuschalten.

8. Verfahren nach einem der Ansprüche 1 bis 7, **dadurch gekennzeichnet, dass** eine oder mehrere der Schallwelle(n) (41) eingebracht werden, indem einer oder mehrere der Schallwandler (44) an Seitenflächen des Substrats angeordnet werden.

9. Verfahren nach einem der Ansprüche 1 bis 8, **dadurch gekennzeichnet, dass** in Kombination mit der Manipulation durch die stehende(n) und/oder stationäre(n) Schallwelle(n) (14) eine dielektrophoretische Manipulation der Teilchen und/oder Zellen und/oder Viren in der Mikrokammer oder dem Mikrokanal (11) durchgeführt wird.

10. Verfahren nach Anspruch 9, **dadurch gekennzeichnet, dass** die dielektrophoretische Manipulation in Bezug auf eine laminare Strömung der Teilchen und/oder Zellen und/oder Viren in der Mikrokammer oder dem Mikrokanal (11) stromabwärts eines Manipulationsbereichs durch mindestens eine der stehenden und/oder stationären Schallwelle(n) (14) durchgeführt wird.

11. Verfahren nach Anspruch 10, **dadurch gekennzeichnet, dass** die Teilchen und/oder Zellen und/oder Viren zuerst durch die stehende(n) und/oder stationäre(n) Schallwelle(n) (14) in einer oder mehreren Reihen ausgerichtet werden und dann durch die dielektrophoretische Manipulation eingeschlossen werden.

12. Verfahren nach Anspruch 9, **dadurch gekennzeichnet, dass** die dielektrische Manipulation und die Manipulation durch die stehende(n) und/oder stationäre(n) Schallwelle(n) (14) zur selben Zeit in überlappenden Bereichen des Volumens vorgenommen werden.

13. Verfahren nach einem der Ansprüche 1 bis 12, **dadurch gekennzeichnet, dass** mindestens zwei parallele laminare Strömungen verschiedener Fluide (103, 104) mit den Teilchen und/oder Zellen und/oder Viren in dem Mikrokanal oder der Mikrokammer (11) erzeugt werden, wobei die Teilchen und/oder Zellen und/oder Viren von einer ersten der laminaren Strömungen in eine zweite der laminaren Strömungen umgeschaltet werden, indem die Frequenz einer oder mehrerer der Schallwelle(n) (41) verschoben oder umgeschaltet wird oder indem eine dielektrophoretische Kraft angewendet wird.

14. Verfahren nach Anspruch 13, **dadurch gekennzeichnet, dass** die Teilchen und/oder Zellen und/oder Viren, welche in die zweite laminare Strömung umgeschaltet werden, in einem definierten Bereich in der zweiten laminaren Strömung eingeschlossen werden, indem mittels einer stehenden und/oder stationären Schallwelle (14) über die zweite laminare Strömung hinweg eine Barriere (109) für die Teilchen und/oder Zellen und/oder Viren erzeugt wird.

15. Verfahren nach Anspruch 13, **dadurch gekennzeichnet, dass** die Teilchen und/oder Zellen und/oder Viren, welche in die zweite laminare Strömung umgeschaltet werden, in einem definierten Bereich in der zweiten laminaren Strömung eingeschlossen werden, indem mittels dielektrophoretischer Kräfte über die zweite laminare Strömung hinweg eine Barriere (109) für die Teilchen und/oder Zellen und/oder Viren erzeugt wird.

16. Verfahren nach Anspruch 13, **dadurch gekennzeichnet, dass** die Teilchen und/oder Zellen und/oder Viren, welche in die zweite laminare Strömung umgeschaltet werden, in einem definierten Bereich in der zweiten laminaren Strömung eingeschlossen werden, indem eine Strömungsrichtung der zweiten laminaren Strömung periodisch umgekehrt wird.

17. Vorrichtung zum nicht intrusiven Manipulieren suspendierter Teilchen und/oder Zellen und/oder Viren, welche ein Substrat mit mindestens einer integrierten Mikrokammer oder einem integrierten Mikrokanal (11) umfasst, wobei die Mikrokammer oder der Mikrokanal (11) mindestens eine untere Wand (12b) sowie seitliche Wände (12c, 12d) aufweist, und mit mindestens einem Schallwandler (44), der dafür eingerichtet ist, von außerhalb des Substrats eine Schallwelle (41) seitlich in ein inneres Volumen der Mikrokammer oder des Mikrokanals (11) einzubringen,
**dadurch gekennzeichnet, dass** einer oder mehrere der Schallwandler (44) auf einem Schallbrechungselement (43) angebracht sind, welches an einer oberen und/oder einer unteren Fläche des Substrats angebracht ist, wobei das Brechungselement so ausgebildet ist, dass die Schallwelle(n) (41) in einer Richtung in das Substrat eingekoppelt wird (werden), die sich von der Richtung einer Flächennormalen einer oberen Fläche oder einer unteren Fläche des Substrats unterscheidet.

18. Vorrichtung nach Anspruch 17, **dadurch gekennzeichnet, dass** die Schallwandler (44) an verschiedenen Positionen des Substrats angeordnet sind.

19. Vorrichtung nach Anspruch 18, **dadurch gekennzeichnet, dass** die Schallwandler (44) an verschiedenen Seiten des Substrats angeordnet sind.

20. Vorrichtung nach einem der Ansprüche 17 bis 19,
**dadurch gekennzeichnet, dass** der eine oder die mehreren der Schallwandler (44) an einer oder mehreren Seitenflächen des Substrats angebracht sind.

21. Vorrichtung nach einem der Ansprüche 17 bis 20,
**dadurch gekennzeichnet, dass** es sich bei den Brechungselementen (43) um keilförmige oder prismenförmige Elemente handelt.

22. Vorrichtung nach einem der Ansprüche 17 bis 21,
**dadurch gekennzeichnet, dass** der Schallwandler (44) außerhalb eines geraden Strahlenwegs durch das Volumen und die untere Wand hindurch angeordnet ist.

23. Vorrichtung nach einem der Ansprüche 17 bis 22, **dadurch gekennzeichnet, dass** in die untere Wand (12b) und/oder in eine obere Wand (12a) Elektroden (111a, 111b) integriert sind, welche eine dielektrophoretische Manipulation der Teilchen und/oder Zellen und/oder Viren in der Mikrokammer oder dem Mikrokanal (11) in Kombination mit der Manipulation durch (eine) stehende und/oder stationäre Schallwelle(n) (14) ermöglichen, die durch die ein oder mehreren Schallwandler (44) erzeugt werden.

24. Vorrichtung nach Anspruch 23, **dadurch gekennzeichnet, dass** die Elektroden (111a, 111b) in Bezug auf eine laminare Strömung der Teilchen und/oder Zellen und/oder Viren in der Mikrokammer oder dem Mikrokanal (11) außerhalb eines oder mehrerer Bereiche der Manipulation durch die stehende(n) und/oder stationäre(n) Schallwelle(n) (14) angeordnet sind.

25. Vorrichtung nach Anspruch 23, **dadurch gekennzeichnet, dass** die Elektroden (111a, 111b) in verschiedenen Bereichen der Mikrokammer oder des Mikrokanals (11) angeordnet sind, welche sich mit Bereichen der Manipulation durch die stehende(n) und/oder stationäre(n) Schallwelle(n) (14) auf einer Längsachse der Mikrokammer oder des Mikrokanals (11) abwechseln.

26. Vorrichtung nach Anspruch 23, **dadurch gekennzeichnet, dass** die Elektroden (111a, 111b) in einem oder mehreren Bereichen angeordnet sind, die von der (den) stehenden und/oder stationären Schallwelle(n) (14) beeinflusst werden.

27. Vorrichtung nach einem der Ansprüche 23 bis 26, **dadurch gekennzeichnet, dass** die Elektroden (111a, 111b) so angeordnet sind, dass sie eine oder mehrere Fallen für die Teilchen und/oder Zellen und/oder Viren über dielektrophoretische Manipulation bilden können.

28. Vorrichtung nach einem der Ansprüche 17 bis 27, **dadurch gekennzeichnet, dass** das Substrat zumindest in dem Bereich einer oberen Wand (12a) und der unteren Wand (12b) der Mikrokammer oder des Mikrokanals (11) aus einem optisch transparenten Material ist.

29. Vorrichtung nach Anspruch 28, **dadurch gekennzeichnet, dass** die obere Wand (12a) und die untere Wand (12b) der Mikrokammer oder des Mikrokanals (11) eine Dicke aufweisen, welche für eine Beobachtung der Teilchen und/oder Zellen und/oder Viren in dem Volumen die Anwendung der Transmissionsmikroskopie mit hoher numerischer Apertur ermöglicht.

## Revendications

1. Procédé de manipulation non intrusive de particules et/ou cellules et/ou virus suspendus qui sont apportés à une micro-chambre ou à un micro-canal (11) d'un substrat, ladite micro-chambre ou ledit micro-canal (11) comportant au moins une paroi de fond (12b) ainsi que des parois latérales (12c, 12d), au moins une onde acoustique (41) étant appliquée via au moins un transducteur acoustique (44) latéralement à un volume intérieur de ladite micro-chambre ou dudit micro-canal (11), une fréquence de ladite onde acoustique (41) étant sélectionnée de manière à générer une onde acoustique fixe et/ou stationnaire (14) dans ledit volume,
**caractérisé en ce que**
ladite au moins une onde acoustique (41) est appliquée via au moins un transducteur acoustique (44) depuis l'extérieur dudit substrat au volume intérieur de ladite micro-chambre ou dudit micro-canal (11), une ou plusieurs desdites ondes acoustiques (41) étant appliquées en utilisant un élément réfractaire acoustique (43) entre un ou plusieurs desdits transducteurs acoustiques (44) et ledit substrat, ledit élément réfractaire (43) étant formés de manière à lancer ladite(lesdites) onde(s) acoustique(s) (41) dans ledit substrat dans un sens différent d'un sens normal de surface d'une surface de dessus ou d'une surface de fond dudit substrat.

2. Procédé selon la revendication 1,
**caractérisé en ce que** ledit élément réfractaire (43) est un élément en forme de coin ou en forme de prisme.

3. Procédé selon la revendication 1 ou 2,
**caractérisé en ce qu'**un ou plusieurs desdits éléments réfractaires (43) sont rattachés à la surface de dessus et/ou la surface de fond dudit substrat.

4. Procédé selon l'une quelconque des revendications 1 à 3,
**caractérisé en ce que** ledit au moins un transducteur acoustique (44) est disposé à l'extérieur d'un parcours optique rectiligne traversant ledit volume et ladite paroi de fond (12b).

5. Procédé selon l'une quelconque des revendications 1 à 4,
**caractérisé en ce que** plusieurs ondes acoustiques sont appliquées latéralement via plusieurs transducteurs acoustiques (44) à des zones différentes et/ou dans différents sens de ladite micro-chambre ou dudit micro-canal (11) afin de générer des ondes acoustiques fixes et/ou stationnaires (14) dans ledit volume, ce qui permet de réaliser une manipulation identique ou différente dans des zones différentes ou identiques de la micro-chambre ou du micro-canal (11).

6. Procédé selon l'une quelconque des revendications 1 à 5,
**caractérisé en ce que** la fréquence d'une ou plusieurs desdites ondes acoustiques (41) est sélectionnée de manière à former un ou plusieurs noeuds ou antinoeuds de ladite(desdites) onde(s) acoustique(s) et/ou stationnaire(s) (14) entre les parois latérales opposées de ladite micro-chambre ou dudit micro-canal (11) .

7. Procédé selon l'une quelconque des revendications 1 à 6,
**caractérisé en ce que** la fréquence d'une ou plusieurs desdites ondes acoustiques (41) est modifiée de manière à permuter entre différents modèles de noeuds des ondes acoustiques fixes et/ou stationnaires (14).

8. Procédé selon l'une quelconque des revendications 1 à 7,
**caractérisé en ce qu'**une ou plusieurs desdites ondes acoustiques (41) sont appliquées en disposant un ou plusieurs desdits transducteurs acoustiques (44) sur les surfaces latérales dudit substrat.

9. Procédé selon l'une quelconque des revendications 1 à 8,
**caractérisé en ce que**, en combinaison avec la manipulation par ladite(lesdites) onde(s) acoustique(s) et/ou stationnaire(s) (14), une manipulation diélectrophorétique desdits particules et/ou cellules et/ou virus dans ladite micro-chambre ou ledit micro-canal (11) est réalisée.

10. Procédé selon la revendication 9,
**caractérisé en ce que** ladite manipulation diélectrophorétique est réalisée en aval d'une zone de manipulation par au moins une de ladite(desdites) onde(s) acoustique(s) et/ou stationnaire(s) (14) par rapport à un flux laminaire desdits particules et/ou cellules et/ou virus dans ladite micro-chambre ou ledit micro-canal (11).

11. Procédé selon la revendication 10,
**caractérisé en ce que** lesdits particules et/ou cellules et/ou virus sont d'abord alignés en une ou plusieurs rangées par ladite(lesdites) onde(s) acoustique(s) et/ou stationnaire(s) (14) puis piégées par ladite manipulation électrophorétique.

12. Procédé selon la revendication 9,
**caractérisé en ce que** ladite manipulation diélectrophorétique et ladite manipulation par ladite(lesdites) onde(s) acoustique(s) et/ou stationnaire(s) (14) sont réalisées dans des zones se chevauchant dudit volume en même temps.

13. Procédé selon l'une quelconque des revendications 1 à 12,
**caractérisé en ce qu'**au moins deux flux laminaires parallèles de fluides différents (103, 104) contenant lesdits particules et/ou cellules et/ou virus sont générés dans ledit micro-canal ou ladite micro-chambre (11), lesdits particules et/ou cellules et/ou virus étant permutés d'un premier desdits flux laminaires vers un second desdits flux laminaires en modifiant ou en permutant la fréquence d'une ou plusieurs desdites ondes acoustiques (41) ou en appliquant une force diélectrophorétique.

14. Procédé selon la revendication 13,
**caractérisé en ce que** lesdits particules et/ou cellules et/ou virus qui sont permutés vers le second flux laminaire sont piégés dans une zone définie dudit second flux laminaire en générant une barrière (109) pour lesdits particules et/ou cellules et/ou virus à travers ledit second flux laminaire au moyen d'une onde acoustique fixe et/ou stationnaire (14).

15. Procédé selon la revendication 13,
**caractérisé en ce que** lesdits particules et/ou cellules et/ou virus qui sont permutés vers le second flux laminaire sont piégés dans une zone définie dudit second flux laminaire en générant une barrière (109) pour lesdits particules et/ou cellules et/ou virus à travers ledit second flux laminaire au moyen de forces diélectrophorétiques.

16. Procédé selon la revendication 13,
**caractérisé en ce que** lesdits particules et/ou cellules et/ou virus qui sont permutés vers le second flux laminaire sont piégés dans une zone définie dudit second flux laminaire en inversant périodiquement un sens de flux dudit second flux laminaire.

17. Dispositif de manipulation non intrusive de particules et/ou cellules et/ou virus suspendus, comprenant un substrat comportant au moins une micro-chambre ou un micro-canal intégré (11), ladite micro-chambre ou ledit micro-canal (11) comportant au moins une paroi de fond (12b) ainsi que des parois latérales (12c, 12d) et au moins un transducteur acoustique (44) disposé de manière à appliquer une onde acoustique (41) depuis l'extérieur dudit substrat latéralement à un volume intérieur de ladite micro-chambre ou dudit micro-canal (11),
**caractérisé en ce qu'**un ou plusieurs desdits transducteurs acoustiques (44) sont montés sur un élément réfractaire acoustique (43) rattaché à une surface de dessus et/ou une surface de fond dudit substrat, ledit élément réfractaire (43) étant formé de manière à lancer ladite(lesdites) onde(s) acoustique(s) (41) dans ledit substrat dans un sens différent d'un sens normal de surface d'une surface de dessus ou d'une surface de fond dudit substrat.

18. Dispositif selon la revendication 17,
**caractérisé en ce que** lesdits transducteurs acoustiques (44) sont disposés à des positions différentes dudit substrat.

19. Dispositif selon la revendication 18,
**caractérisé en ce que** lesdits transducteurs acoustiques (44) sont disposés sur des faces différentes dudit substrat.

20. Dispositif selon l'une quelconque des revendications 17 à 19,
**caractérisé en ce qu'**un ou plusieurs desdits transducteurs acoustiques (44) sont montés sur une ou plusieurs surfaces latérales dudit substrat.

21. Dispositif selon l'une quelconque des revendications 17 à 20,
**caractérisé en ce que** lesdits éléments réfractaires (43) sont des éléments en forme de coin ou en forme de prisme.

22. Dispositif selon l'une quelconque des revendications 17 à 21,
**caractérisé en ce que** ledit transducteur acoustique (44) est disposé à l'extérieur d'un parcours optique rectiligne traversant ledit volume et ladite paroi de fond.

23. Dispositif selon l'une quelconque des revendications 17 à 22,
**caractérisé en ce que**, dans ladite paroi de fond (12b) et/ou dans une paroi de dessus (12a), sont intégrées des électrodes (111a, 111b) permettant une manipulation diélectrophorétique desdits particules et/ou cellules et/ou virus dans ladite micro-chambre ou ledit micro-canal (11) en combinaison avec la manipulation par une(des) onde(s) acoustique(s) fixes et/ou stationnaires (14) générée(s) par lesdits un ou plusieurs transducteurs acoustiques (44).

24. Dispositif selon la revendication 23,
**caractérisé en ce que** lesdites électrodes (111a, 111b) sont disposées à l'extérieur d'une ou plusieurs zones de manipulation par ladite(lesdites) onde(s) acoustique(s) et/ou stationnaire(s) (14) par rapport à un flux laminaire desdits particules et/ou cellules et/ou virus dans ladite micro-chambre ou ledit micro-canal (11).

25. Dispositif selon la revendication 23,
**caractérisé en ce que** lesdites électrodes (111a, 111b) sont disposées dans différentes zones de ladite micro-chambre ou dudit micro-canal (11) alternant avec des zones de manipulation par ladite(lesdites) onde(s) acoustique(s) et/ou stationnaire(s) (14) sur un axe longitudinal de ladite micro-chambre ou dudit micro-canal (11).

26. Dispositif selon la revendication 23,
**caractérisé en ce que** lesdites électrodes (111a, 111b) sont disposées dans une zones influencées par ladite(lesdites) onde(s) acoustique(s) et/ou stationnaire(s) (14).

27. Dispositif selon l'une quelconque des revendications 23 à 26,
**caractérisé en ce que** lesdites électrodes (111a, 111b) sont disposées de manière à pouvoir former un ou plusieurs pièges pour lesdits particules et/ou cellules et/ou virus via manipulation diélectrophorétique.

28. Dispositif selon l'une quelconque des revendications 17 à 27,
**caractérisé en ce que** ledit substrat est fait d'une matière optiquement transparente au moins dans la zone d'une paroi de dessus (12a) et de ladite paroi de fond (12b) de ladite micro-chambre ou dudit micro-canal (11).

29. Dispositif selon la revendication 28,
**caractérisé en ce que** ladite paroi de dessus (12a) et ladite paroi de fond (12b) de ladite micro-chambre ou dudit micro-canal (11) ont une épaisseur permettant l'utilisation d'une microscopie de transmission à grande ouverture numérique pour l'observation desdits particules et/ou cellules et/ou virus dans ledit volume.
